# EUROPEAN PATENT SPECIFICATION

(11) **EP 3 119 293 B1**
(45) Date of publication and mention of the grant of the patent: **13.05.2020**
(21) Application number: 15715493.1
(22) Date of filing: 17.03.2015
(51) Int. Cl.: A61B 17/322

(54) **DERMATOME, BLADE AND ASSEMBLY OF A DERMATOME AND A BLADE**
DERMATOM, KLINGE UND ANORDNUNG EINES DERMATOMS UND EINER KLINGE
DERMATOME, LAME ET ENSEMBLE D'UN DERMATOME ET D'UNE LAME

(30) Priority: 21.03.2014 NL 2012487; 09.01.2015 NL 2014106
(43) Date of publication of application: 25.01.2017
(73) Proprietor: Humeca Holding B.V., 7544 DB Enschede (NL)
(72) Inventor: HAAGSMA-VAN DER KOUWE, Daniëlle, NL-7623 KW Borne (NL)
(74) Representative: Bartelds, Erik
(86) International application number: PCT/NL2015/050171
(87) International publication number: WO 2015/142172

(56) References cited:
- GB-A- 645 289
- GB-A- 1 231 083
- US-A- 3 934 591
- US-A1- 2009 157 095
- US-A1- 2010 211 173

## Description

### Background

US 2009/157095 A1 discloses a dermatome comprising: a blade holder, a guiding element for guiding an oscillating movement of a blade and a spacer for defining a cutting gap.US 2010/211173 A1 discloses a cartilage cutter having a curved blade.

The invention relates to a dermatome, comprising:
- a blade holder for holding a blade, said blade holder comprising at least one guiding element for guiding an oscillating movement of a said blade, and
- at least one spacer that is spaced apart from said blade holder for defining a cutting gap between a said blade held by said blade holder and said at least one spacer,

The invention further relates to a blade suitable for use in a dermatome. In particular the invention relates to dermatome blades. More in particular the invention relates to a dermatome blade apparently intended to be used in a dermatome according to the invention.

The invention also relates to an assembly of a dermatome and a blade.

Such a dermatome and blade are known perse. A dermatome is used for harvesting skin graft. The width of the skin graft is limited to the width of the skin that is in contact with the blade of the dermatome. For some surfaces, for example a thighbone, this width may be relatively large, while for other surfaces, such as the head where the skin is relatively tight to the skull, this width may be relatively small. In using the skin grafts a relatively large width may be preferred.

It is an object of the invention to be able to increase the width of a harvested skin graft by increasing the width of the contact area of the skin with the blade on at least some surfaces of a human being.

This object is achieved by a dermatome according to the preamble that is characterized in that said at least one guiding element and said at least one spacer define a curved path in their longitudinal direction. The invention is further defined by the features of claim 11.

At least in a use position of the blade the blade is also curved. In particular a cutting edge of said blade is curved at least in said use position.

As a result of the curvature of the path defined by the at least one guiding element, the at least one spacer and at least in use said blade, the width of the contact area of the skin with the blade may be relatively large, such that the harvested skin graft has a relatively large width. For example for the head of a person, or other curved surfaces, in particular curved surfaces where the skin is relatively tight to the bone the contact area and thereby the width of the harvested skin may be increased with respect to a straight or uncurved blade and dermatome.

The curvature of the defined curved path may be any suitable curvature, for example, but not limited thereto, circular or elliptical. The curvature may optionally be variable over its length or may optionally be asymmetric. In case of a circular curvature the radius may be chosen as desired, for example in accordance with a curvature of a particular surface of a human being or an animal. Various dermatomes with various curvatures and/or radii may be provided, such that for a surface of a human being or animal a suitable dermatome with a suitable curvature may be selected. The radius may for example be chosen between 50 - 300 mm, in particular between 100 - 200 mm.

It is noted that said oscillating movement of the blade is in a direction parallel to a longitudinal direction of said blade, i.e. in a direction parallel to the cutting edge of the blade.

It is noted that the defined path of the at least one guiding element and the at least one spacer are curved in their longitudinal direction.

For example, three or more spaced apart guiding elements may be provided that are arranged such that together they define a curved path.

For example, three or more spaced apart spacers may be provided that are arranged such that together they define a curved path.

By increasing the number of spaced apart guiding elements or spacers the accuracy of the thereby defined curved path may be increased, as the spaced apart guiding elements or spacers will then more and more resemble a continuous curve.

In an embodiment of the invention said guiding element has a guiding surface and said spacer has a spacer surface, wherein said guiding surface and said spacer surface are curved.

Such a curved guiding surface and curved spacer surface define an accurate curved path.

In this embodiment the defined curved path of the at least one guiding element and the at least one spacer are the curved guiding surface and the curved spacer surface.

It is noted that said guiding element and/or said spacer surface may be either (substantially) continuous or discontinuous surfaces.

In an embodiment of the dermatome according to the invention, said dermatome comprises at least one locking means for locking a said blade to said blade holder, said at least one locking means defining a curved path.

The defined curved path of the at least one locking means is arranged opposite to said curved path defined by said at least one guiding element, thereby forming a curved space for containing said blade. If the blade is a flexible blade, it is bended in its curved shape by said curved paths of said at least one guiding element and said at least one locking element.

For example, three or more spaced apart locking elements may be provided that are arranged such that together they define a curved path.

By increasing the number of spaced apart locking elements the accuracy of the thereby defined curved path may be increased, as the spaced apart locking elements will then more and more resemble a continuous curve.

In an embodiment of the invention said locking means has a locking surface, wherein said locking surface is curved.

Such a curved locking surface defines an accurate curved path.

In this embodiment the defined curved path of the at least one locking means is the curved locking surface.

It is noted that said locking surface may be either a (substantially) continuous or discontinuous surface.

In another embodiment of the dermatome according to the invention the curvatures of the defined curved paths of the at least one guiding element, the at least one spacer and/or the at least one locking means are substantially equal.

An advantage of the curvatures of the defined curved paths of the at least one guiding element, the at least one spacer and/or the at least one locking means being substantially equal is that the thickness of the harvested skin graft may be substantially uniform over its width if a blade is chosen with a same curvature or if a flexible blade is bended to the same curvature between the guiding element and the spacer.

In particular, the curvatures of the guiding element, the spacer surface and/or the locking surface may be substantially equal.

Practically said dermatome comprises a driving means for driving a said blade in said oscillating movement with respect to said at least one guiding element or guiding surface.

Said driving means may be any known, suitable driving means. Said driving means may for example be, but is not limited thereto, electrically powered, for example by an optionally releasable connected battery or by a connection to the mains electricity net, or for example by compressed air.

In yet another embodiment of the dermatome according to the invention said driving means comprises a first connecting means for connecting to a respective second connecting means of said blade, which first connecting means is drivable in said oscillating movement with respect to said at least one guiding element or guiding surface.

For example, said first connecting means may comprise a protrusion or a recess that are arranged for being inserted in or to receive a corresponding recess or protrusion of the second connecting means.

In yet another embodiment of the dermatome according to the invention said at least one guiding element or guiding surface comprises guiding means for guiding the oscillating movement of a said blade with respect to said at least one guiding element or guiding surface.

Said guiding means may for example comprise a stop (surface) suitable for receiving said blade in an abutting position.

In use said blade abuts to said stop (surface) and is thereby held in a correct, aligned position with respect to the at least one spacer or spacer surface. Said stop (surface) may for example be an upstanding element or edge connecting to a longitudinal end zone of said at least one guiding element or guiding surface. Said edge may be continuous over its end or may be interrupted.

Alternatively or additionally said guiding means may comprise guiding cams or guiding recesses that are arranged for being inserted in or to receive corresponding guiding recesses or guiding cams of a said blade. Such guiding cams and recesses may also help positioning said blade with respect to the at least one guiding element or guiding surface at inserting said blade.

In yet another embodiment of the dermatome according to the invention said at least one spacer is movably mounted with respect to said guiding element, such that the width of the cutting gap is adjustable.

Such a movable spacer offers the advantage of being able to adjust the width of the cutting gap and thereby the thickness of the skin graft.

Said blade may have a curved shape adapted to the curved path defined by the at least one guiding element. Such a blade is permanently fixed in its curved shape. Although such a blade may be used for one curvature only, such that optionally different types of blades having different curvatures need to be provided, such a curved blade may be easier to insert in said blade holder.

Said blade may comprise second connecting means for connecting to the first connecting means of the driving means.

Said blade may comprise recesses or guiding cams that are arranged for being inserted in or to receive corresponding guiding cams or recesses of the guiding means. Such recesses or guiding cams may help position the blade in a correct position and/or may guide the oscillating movement of the blade.

The invention further relates to an assembly of a dermatome according to the invention comprising any one or any combination of the above described features and a blade comprising any one or any combination of the above described features, wherein said blade is held by the blade holder of the dermatome, or a dermatome blade that flexible such that it is bendable in a curved shape between said at least one guiding element and said at least one locking means, wherein the blade is at least flexible in its longitudinal direction, said blade being held by the blade holder of the dermatome.

In an embodiment of the assembly having the flexible dermatome blade, the flexible blade may further comprise second connecting means for connecting to the first connecting means of the driving means.

In another embodiment of the assembly having the flexible dermatome blade, the flexible blade may further comprise recesses or guiding cams that are arranged to receive or for being inserted in corresponding guiding cams or recesses of the guiding means
The invention will be further elucidated with reference to figures shown in a drawing, in which:
- Figure 1 is a perspective view of a dermatome according to an embodiment of the invention in use;
- Figure 2 is a perspective view of the other side of the dermatome of figure 1;
- Figure 3 is a front view of the dermatome of figure 1;
- Figure 4 is the perspective view of figure 2 in a position for inserting a blade,
- Figure 5 is the perspective view of figure 4 with an inserted blade,
- Figures 6 and 7 show two types of blades suitable to be used with the dermatome of figure 1.

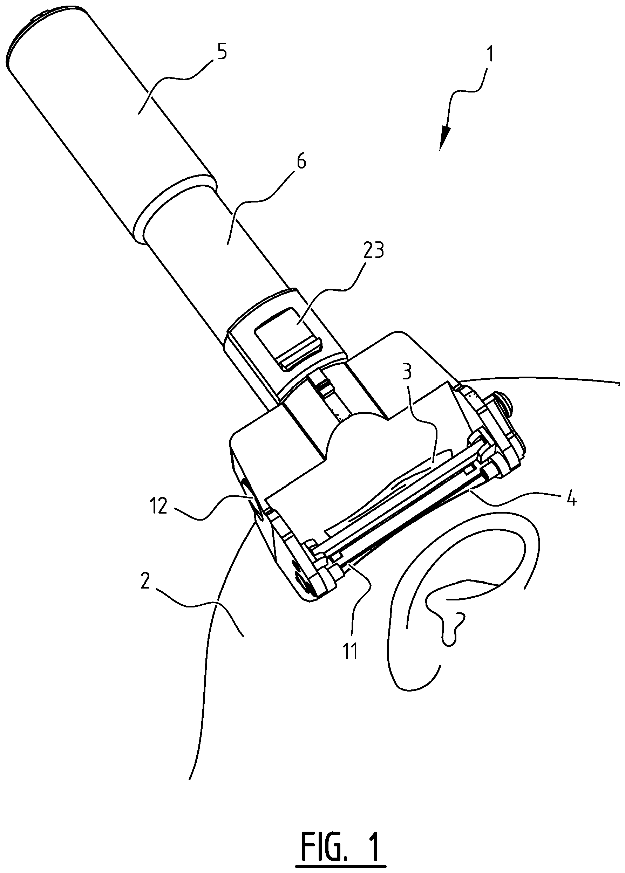

Figures 1 - 7 show a dermatome 1 according to an embodiment of the invention. Similar elements are denoted by similar reference numerals.

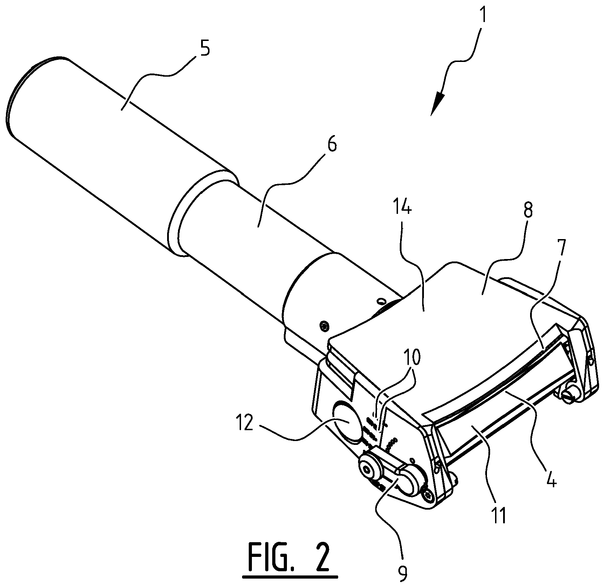

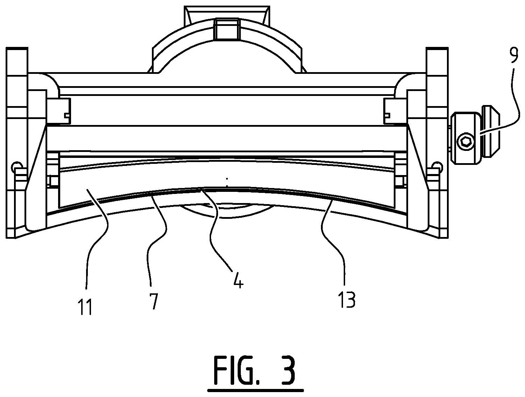

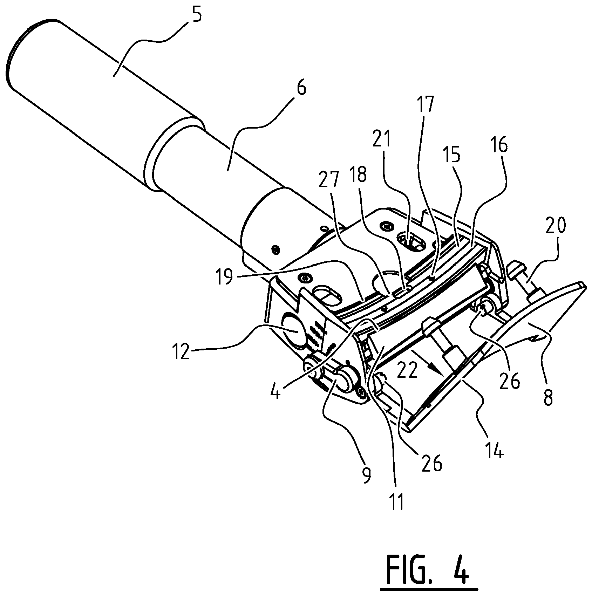

In figure 1, the dermatome 1 is shown in use for harvesting skin graft 3 from a head 2 of a human being. As is clearly shown in this figure 1, a spacer surface 4 of a spacer element 11 is curved, such that it corresponds to the curved shape of the head 2, in this example. As a result of this curvature the contact area with the head 2 is relatively large as compared to a straight or uncurved spacer element and thereby the width of the skin graft 3 is also relatively large. The dermatome 1 has a grip 5 containing a battery, which grip 5 is connected to a shaft 6 of the dermatome 1. A button 23 is shown for activating the dermatome 1. To the sides of the dermatome 1 two buttons 12 are provided, which may be pressed simultaneously for opening a locking element 14 of the dermatome 1. Figure 2 shows the other side of the dermatome 1. The dermatome has a contact surface 8 that may be put in contact with the skin of a human being or animal. This contact surface 8 is curved to correspond to a curved surface of the human being. The contact surface 8 is part of the locking element 14 for locking the blade 7 to the blade holder, see also figure 4. A frontal longitudinal edge of the blade 7 extends slightly beyond the contact surface 8, such that it may contact the skin for cutting the skin. The spacer surface 4 is located at a predetermined distance from the blade 7, thereby defining a cutting gap 13 (see figure 3) between the spacer surface 4 and the blade 7, which defines the thickness of the skin to be harvested. To the side of the dermatome 1 a handle 9 is provided, which is connected to the spacer element 11 for moving the spacer element 11 with respect to a guiding surface 15 (see figure 4) of the dermatome for adjusting the width of the cutting gap. Denoted by number 10 are fixed positions for the handle 9, such that the desired position of the spacer element 11 may easily be selected. Instead of fixed positions the spacer element 11 may be adjustable in a continuous way.

Figure 3 is a frontal view of the dermatome 1. From this figure it is clear that the curvatures of the blade 7 and the spacer surface 4 are substantially equal. In particular the inner radius of the blade 7 is equal to the outer radius of the spacer surface 4. The radius is 160 mm in this example. The small gap 13 located between the blade 7 and the spacer surface 4 has therefore a substantially uniform thickness over its length, such that the thickness of the harvested skin graft is substantially uniform. It is noted that in particular for one position of the adjustable spacer element 11 the small gap 13 has a uniform thickness over its length, for example for a position of the spacer element 11 that is most frequently used. In the other positions of the adjustable spacer element the small gap 13 may have a thickness that is almost or substantially equal over its length.

Figure 4 shows the dermatome 1 in the position of figure 2, wherein the locking element 14 comprising said contact surface 8 is opened to an open position for inserting the blade 7. This shows that the dermatome 1 has a guiding surface 15 with a curvature that corresponds to the curvatures of the blade 7 and spacer surface 4, or will bend the blade 7 in the desired curvature. The locking element 14 has a locking surface 22 with a curvature corresponding to the curvatures of the guiding surface 15 and the spacer surface 4. The locking surface 22 is located opposite to the contact surface 8. The blade 7 may be arranged on the guiding surface 15 for inserting the blade 7 to the blade holder. The guiding surface 15 has a longitudinal groove 16 for decreasing the contact area between the guiding surface 15 and the blade 7 and thereby the friction therebetween. The guiding surface 15 further has two guiding cams 17 and an upstanding edge 19. A protrusion 18 of a driving means is also provided, which protrusion 18 is drivable in an oscillating movement with respect to the guiding surface 15 in the longitudinal direction thereof. Said protrusion 18 is oscillating in a elongated slot or recess 27 extending in the longitudinal direction of the guiding surface 15. The movement of the protrusion 18 is electrically driven by a motor electrically powered by said battery. After inserting a blade 7 the locking element 8 may be closed for locking the blade 7 to the blade holder by inserting locking pins 20 of the locking element 14 in respective locking recesses 21 of an opposing surface of the dermatome 1. As described above, two buttons 12 may be pressed simultaneously for releasing the locking pins 20 from the locking recesses 21 and for opening the locking element 14. The locking element 14 is pivotable between its open position shown in figure 4 and its closed position shown in figure 2 via hinges 26.

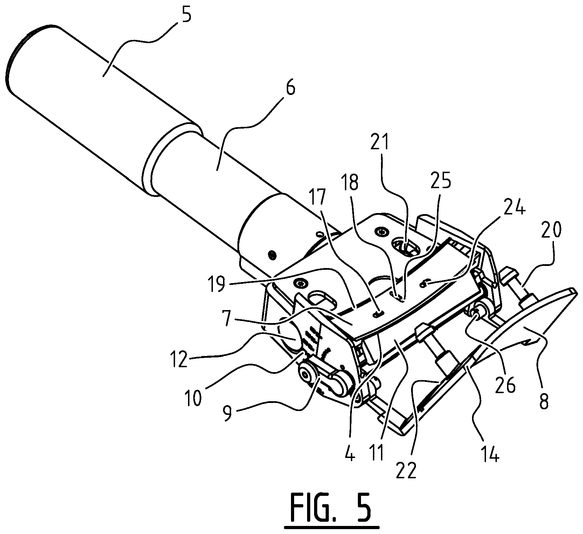

Figure 5 shows the blade 7 in inserted position. This shows that the blade 7 has two elongated slots or recesses 24 extending in the longitudinal direction of the blade 7 and an elongated slot or recess 25 extending in a transverse direction of the blade 7. The recesses 24 are arranged for receiving the guiding cams 17 of the guiding surface 15 and the recess 25 is arranged for receiving the protrusion 18 of the driving means. The recesses 24 have a length such that the guiding cams 17 do not reach the end zones thereof during the oscillating movement of the blade 7. The guiding cams 17 therefore, in at least this embodiment, do not limit the maximum oscillating movement of the blade 7. The guiding cams 17 provide an easy alignment of the blade 7 to the guiding surface 15, and may therefore also be seen as alignment or positioning cams. A rear end zone of the blade 7 abuts against the upstanding edge 19, which edge 19 holds the blade 7 in a correct position during use of the dermatome 1. The recess 25 extends over a certain length in the transverse direction for allowing some movement of the protrusion 18 in the transverse direction and/or some movement of the blade 7, such that the blade 7 may easily be inserted and may abut said edge 19.

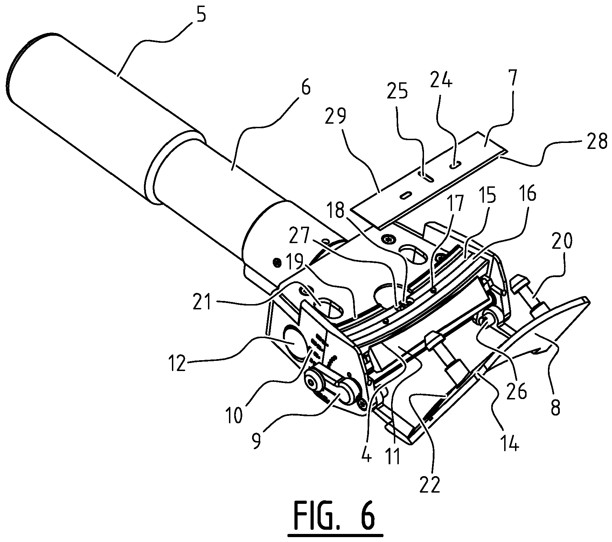

Figure 6 shows a first type of blade 7. The blade 7 is a straight, flexible blade, such that it is bendable in a curved position. The blade is at least flexible in the longitudinal direction thereof. After insertion of the blade to the guiding surface 15 and closing the locking element 14, the blade 7 is bended to its curved position therebetween. The curvature of the blade 7 is thereby determined by the curvatures of the guiding surface 15 and locking surface 22. For providing the blade 7 flexibility the blade 7 may for example be made of a flexible material an/or may be relatively thin. As is shown, the blade 7 comprises two recesses 24 and one recess 25. A front longitudinal end zone 28 is a cutting zone of the blade 7 and the rear longitudinal end 29 abuts said edge 19.

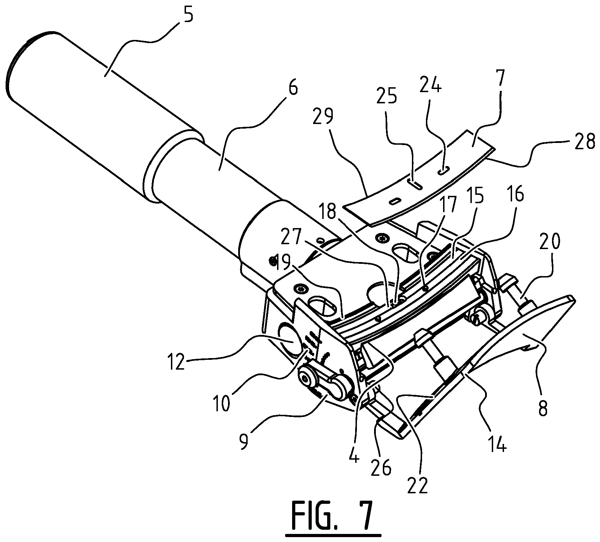

Figure 7 shows a blade 7 that is permanently curved. Said blade may have any desired curvature. Various blades 7 with various curvatures may optionally be provided, such that for harvesting skin graft from a particular surface a blade 7 with a suitable curvature may be chosen, also in accordance with a curvature of at least the guiding surface 15 and spacer surface 4 of the dermatome 1. As is shown, the blade 7 comprises two recesses 24 and one recess 25. A front longitudinal end zone 28 is a cutting zone of the blade 7 and the rear longitudinal end 29 abuts said edge 19.

It is noted that the invention is not limited to the shown embodiments but also extends to variants within the scope of the appended claims.

For example, it is clear for the skilled person that the blade may have a protrusion and guiding cams, and said receiving surface may have a drivable recess and recesses for receiving the guiding cams.

Also, it is clear that the various curvatures of the guiding surface, the spacer surface, the locking surface and the blade may be any desired curvature and are not limited to the circular curvatures that are shown in the figures.

It is also clear for the skilled person that instead of a guiding surface and/or spacer surface and/or a locking surface a plurality of guiding elements and/or spacers and/or locking elements may be provided that define a curved path.

The grip may alternatively comprise a connecting means for connecting to the mains electricity grid instead of accommodating a battery.

For inserting the blade to the blade holder it may optionally be positioned on the locking surface.

## Claims

1. Dermatome (1), comprising:
- a blade holder for holding a blade, said blade holder comprising at least one guiding element for guiding an oscillating movement of a said blade (7), and
- at least one spacer (11) that is spaced apart from said blade holder for defining a cutting gap (13) between a said blade (7) held by said blade holder and said at least one spacer,
**characterized in that**
said at least one guiding element and said at least one spacer (11) define a curved path in their longitudinal direction.

2. Dermatome (1) according to claim 1, comprising at least one locking means (14) for locking a said blade to said blade holder, said at least one locking means defining a curved path.

3. Dermatome (1) according to claim 1 or 2, wherein the curvatures of the defined curved paths of the at least one guiding element, the at least one spacer (11) and/or the at least one locking means (14) are substantially equal.

4. Dermatome (1) according to any of the preceding claims, comprising a driving means for driving a said blade (7) in said oscillating movement with respect to said at least one guiding element.

5. Dermatome (1) according to claim 4, wherein said driving means comprises a first connecting means for connecting to a respective second connecting means of said blade, which first connecting means is drivable in said oscillating movement with respect to said at least one guiding element, optionally wherein said first connecting means comprise a protrusion (18) or a recess that are arranged for being inserted in or to receive a corresponding recess (25) or protrusion of the second connecting means.

6. Dermatome (1) according to any of the preceding claims, wherein said at least one guiding element comprises guiding means for guiding the oscillating movement of a said blade with respect to said at least one guiding element.

7. Dermatome (1) according to claim 6, wherein said guiding means comprise a stop surface (19) suitable for receiving said blade (7) in an abutting position.

8. Dermatome (1) according to claim 6 or 7, wherein said guiding means comprise guiding cams (17) or guiding recesses that are arranged for being inserted in or to receive corresponding guiding recesses (24) or guiding cams of a said blade (7).

9. Dermatome (1) according to any of the preceding claims, wherein said at least one spacer (11) is movably mounted with respect to said at least one guiding element, such that the width of the cutting gap is adjustable.

10. Dermatome (1) according to any of the preceding claims 1 - 9, wherein:
- said guiding element has a guiding surface (15) and said spacer (11) has a spacer surface (4);
- optionally, said locking means (14) has a locking surface (22);
- said guiding surface (15) and said spacer surface (4) are curved; and
- optionally, said locking surface (22) is curved.

11. Dermatome blade (7), suitable for use in a dermatome according to any of claims 1 - 10, said blade (7) having a curved shape adapted to the curved path defined by the at least one guiding element.

12. Dermatome blade (7) according to claim 11, comprising second connecting means (25) for connecting to the first connecting means of the driving means.

13. Dermatome blade (7) according to claim 11 or 12, comprising recesses (24) or guiding cams that are arranged to receive or for being inserted in corresponding guiding cams or recesses of the guiding means.

14. Assembly of a dermatome (1) according to any of claims 1 - 10 and:
a dermatome blade (7) according to any of claims 11 - 13, said blade (7) being held by the blade holder of the dermatome; or
a dermatome blade (7), suitable for use in a dermatome according to any of claims 2 - 10, said blade being flexible such that it is bendable in a curved shape between said at least one guiding element and said at least one locking means (14), wherein the blade is at least flexible in its longitudinal direction, said blade (7) being held by the blade holder of the dermatome (1).

15. Assembly according to claim 14 having the flexible dermatome blade (7), further comprising second connecting means (25) for connecting to the first connecting means of the driving means.

16. Assembly according to claim 13 or 14 having the flexible dermatome blade (7), further comprising recesses (24) or guiding cams that are arranged to receive or for being inserted in corresponding guiding cams or recesses of the guiding means.

## Patentansprüche

1. Dermatom (1), das aufweist:
- einen Klingenhalter zum Halten einer Klinge, wobei der Klingenhalter wenigstens ein Führungselement zum Führen einer Schwingungsbewegung der Klinge (7) aufweist, und
- wenigstens einen Abstandshalter (11), der von dem Klingenhalter beabstandet ist, um eine Schneidlücke (13) zwischen der von dem Klingenhalter gehaltenen Klinge (7) und dem wenigstens einen Abstandhalter zu definieren,
**dadurch gekennzeichnet, dass**
das wenigstens eine Führungselement und der wenigstens eine Abstandshalter (11) in ihrer Längsrichtung einen gekrümmten Weg definieren.

2. Dermatom (1) nach Anspruch 1, das wenigstens eine Sperreinrichtung (14) zum Sperren der Klinge mit dem Klingenhalter aufweist, wobei die wenigstens eine Sperreinrichtung einen gekrümmten Weg definiert.

3. Dermatom (1) nach Anspruch 1 oder 2, wobei die Krümmungen der definierten gekrümmten Wege des wenigstens einen Führungselements, des wenigstens einen Abstandshalters (11) und/oder der wenigstens einen Sperreinrichtung (14) im Wesentlichen gleich groß sind.

4. Dermatom (1) nach einem der vorhergehenden Ansprüche, das eine Antriebseinrichtung zum Antreiben der Klinge (7) in der oszillierenden Bewegung in Bezug auf das wenigstens eine Führungselement aufweist.

5. Dermatom (1) nach Anspruch 4, wobei die Antriebseinrichtung eine erste Verbindungseinrichtung zum Verbinden mit einer jeweiligen zweiten Verbindungseinrichtung der Klinge aufweist, wobei die erste Verbindungseinrichtung in der Schwingungsbewegung in Bezug auf das wenigstens eine Führungselement antreibbar ist, wobei die erste Verbindungseinrichtung optional einen Vorsprung (18) oder eine Vertiefung aufweist, die eingerichtet sind, um in eine entsprechende Vertiefung (25) der zweiten Verbindungseinrichtung eingesetzt zu werden oder einen Vorsprung dieser aufzunehmen.

6. Dermatom (1) nach einem der vorhergehenden Ansprüche, wobei das wenigstens eine Führungselement Führungseinrichtungen zum Führen der Schwingungsbewegung der Klinge in Bezug auf das wenigstens eine Führungselement aufweist.

7. Dermatom (1) nach Anspruch 6, wobei die Führungseinrichtungen eine Anschlagoberfläche (19) aufweisen, die geeignet ist, um die Klinge (7) in einer anliegenden Position aufzunehmen.

8. Dermatom (1) nach Anspruch 6 oder 7, wobei die Führungseinrichtungen Führungsnocken (17) oder Führungsvertiefungen aufweisen, die eingerichtet sind, um in entsprechende Führungsvertiefungen (24) der Klinge (7) eingesetzt zu werden oder ihre Führungsnocken aufzunehmen.

9. Dermatom (1) nach einem der vorhergehenden Ansprüche, wobei der wenigstens eine Abstandshalter (11) in Bezug auf das wenigstens eine Führungselement beweglich montiert ist, so dass die Breite der Schneidlücke einstellbar ist.

10. Dermatom (1) nach einem der vorhergehenden Ansprüche 1 - 9, wobei:
- das Führungselement eine Führungsoberfläche (15) hat und der Abstandshalter (11) eine Abstandshalteroberfläche (4) hat;
- die Sperreinrichtung (14) optional eine Sperroberfläche (22) hat;
- die Führungsoberfläche (15) und die Abstandshalteroberfläche (4) gekrümmt sind; und
- die Sperroberfläche (22) optional gekrümmt ist.

11. Dermatomklinge (7), die für die Verwendung in einem Dermatom nach einem der Ansprüche 1 - 10 geeignet ist, wobei die Klinge (7) eine gekrümmte Form hat, die an den gekrümmten Weg, der durch das wenigstens eine Führungselement definiert wird, angepasst ist.

12. Dermatomklinge (7) nach Anspruch 11, die eine zweite Verbindungseinrichtung (25) zum Verbinden mit der ersten Verbindungseinrichtung der Antriebseinrichtung aufweist.

13. Dermatomklinge (7) nach Anspruch 11 oder 12, die Vertiefungen (24) oder Führungsnocken aufweist, die eingerichtet sind, um entsprechende Führungsnocken der Führungseinrichtungen aufzunehmen oder in deren Vertiefungen eingesetzt zu werden.

14. Anordnung eines Dermatoms (1) nach einem der Ansprüche 1 - 10 und:
eine Dermatomklinge (7) nach einem der Ansprüche 11 - 13, wobei die Klinge (7) von dem Klingenhalter des Dermatoms gehalten wird; oder
eine Dermatomklinge (7), die für die Verwendung in einem Dermatom nach einem der Ansprüche 2 - 10 geeignet ist, wobei die Klinge flexibel ist, so dass sie zwischen dem wenigstens einen Führungselement und der wenigstens einen Sperreinrichtung (14) in eine gekrümmte Form biegbar ist, wobei die Klinge wenigstens in ihrer Längsrichtung flexibel ist, wobei die Klinge (7) durch den Klingenhalter des Dermatoms (1) gehalten wird.

15. Anordnung nach Anspruch 14 mit der flexiblen Dermatomklinge (7), die ferner eine zweite Verbindungseinrichtung (25) zum Verbinden mit der ersten Verbindungseinrichtung der Antriebseinrichtung aufweist.

16. Anordnung nach Anspruch 13 oder 14 mit der flexiblen Dermatomklinge (7), die ferner Vertiefungen (24) oder Führungsnocken aufweist, die eingerichtet sind, um entsprechende Führungsnocken der Führungseinrichtungen aufzunehmen oder in deren Vertiefungen eingesetzt zu werden.

## Revendications

1. Dermatome (1), comprenant:
- un support de lame pour supporter une lame, ledit support de lame comprenant au moins un élément de guidage pour guider un mouvement oscillant de ladite lame (7), et
- au moins un espaceur (11) qui est espacé dudit support de lame pour définir un intervalle de coupe (13) entre ladite lame (7) supportée par ledit support de lame et ledit au moins un espaceur,
**caractérisé en ce que**
ledit au moins un élément de guidage et ledit au moins un espaceur (11) définissent une trajectoire incurvée dans leur direction longitudinale.

2. Dermatome (1) selon la revendication 1, comprenant au moins un moyen de blocage (14) pour bloquer ladite lame sur ledit support de lame, ledit au moins un moyen de blocage définissant une trajectoire incurvée.

3. Dermatome (1) selon la revendication 1 ou 2, dans lequel les courbes des trajectoires incurvées définies de l'au moins un élément de guidage, l'au moins un espaceur (11) et/ou l'au moins un moyen de blocage (14) sont sensiblement égales.

4. Dermatome (1) selon l'une quelconque des revendications précédentes, comprenant un moyen d'entraînement pour entraîner ladite lame (7) dans ledit mouvement oscillant par rapport audit au moins un élément de guidage.

5. Dermatome (1) selon la revendication 4, dans lequel ledit moyen d'entraînement comprend un premier moyen de connexion pour se connecter à un second moyen de connexion respectif de ladite lame, lequel premier moyen de connexion peut être entraîné dans ledit mouvement oscillant par rapport audit au moins un élément de guidage, facultativement dans lequel ledit premier moyen de connexion comprend une saillie (18) ou un évidement qui sont agencés pour être insérés dans ou pour recevoir un évidement (25) ou une saillie correspondant du second moyen de connexion.

6. Dermatome (1) selon l'une quelconque des revendications précédentes, dans lequel ledit au moins un élément de guidage comprend des moyens de guidage pour guider le mouvement oscillant de ladite lame par rapport audit au moins un élément de guidage.

7. Dermatome (1) selon la revendication 6, dans lequel lesdits moyens de guidage comprennent une surface d'arrêt (19) adéquate pour recevoir ladite lame (7) dans une position de butée.

8. Dermatome (1) selon la revendication 6 ou 7, dans lequel lesdits moyens de guidage comprennent des cames de guidage (17) ou des évidements de guidage qui sont agencés pour être insérés dans ou pour recevoir des évidements de guidage (24) ou des cames de guidage correspondants de ladite lame (7).

9. Dermatome (1) selon l'une quelconque des revendications précédentes, dans lequel ledit au moins un espaceur (11) est monté de manière mobile par rapport audit au moins un élément de guidage, de sorte que la largeur de l'intervalle de coupe est ajustable.

10. Dermatome (1) selon l'une quelconque des revendications précédentes 1 à 9, dans lequel:
- ledit élément de guidage présente une surface de guidage (15) et ledit espaceur (11) présente une surface d'espaceur (4);
- facultativement, ledit moyen de blocage (14) présente une surface de blocage (22);
- ladite surface de guidage (15) et ladite surface d'espaceur (4) sont incurvées; et
- facultativement, ladite surface de bocage (22) est incurvée.

11. Lame de dermatome (7), adéquate pour une utilisation dans un dermatome selon l'une quelconque des revendications 1 à 10, ladite lame (7) présentant une forme incurvée adaptée à la trajectoire incurvée définie par l'au moins un élément de guidage.

12. Lame de dermatome (7) selon la revendication 11, comprenant un second moyen de connexion (25) pour se connecter au premier moyen de connexion du moyen d'entraînement.

13. Lame de dermatome (7) selon la revendication 11 ou 12, comprenant des évidements (24) ou des cames de guidage qui sont agencés pour recevoir ou être insérés dans des cames de guidage ou des évidements correspondants des moyens de guidage.

14. Assemblage d'un dermatome (1) selon l'une quelconque des revendications 1 à 10 et:
une lame de dermatome (7) selon l'une quelconque des revendications 11 à 13, ladite lame (7) étant supportée par le support de lame du dermatome; ou
une lame de dermatome (7), adéquate pour une utilisation dans un dermatome selon l'une quelconque des revendications 2 à 10, ladite lame étant flexible de sorte qu'elle peut être coudée dans une forme incurvée entre ledit au moins un élément de guidage et ledit au moins un élément de blocage (14), dans lequel la lame est au moins flexible dans sa direction longitudinale, ladite lame (7) étant supportée par le support de lame du dermatome (1).

15. Assemblage selon la revendication 14 présentant la lame de dermatome flexible (7), comprenant en outre un second moyen de connexion (25) pour se connecter au premier moyen de connexion du moyen d'entraînement.

16. Assemblage selon la revendication 13 ou 14 présentant la lame de dermatome flexible (7), comprenant en outre des évidements (24) ou des cames de guidage qui sont agencés pour recevoir ou pour être insérés dans des cames de guidage ou des évidements correspondants des moyens de guidage.
